# FASCICULE DE BREVET EUROPEEN

(11) **EP 1 403 266 B1**
(45) Date de publication et mention de la délivrance du brevet: **09.04.2014**
(21) Numéro de dépôt: 03292319.5
(22) Date de dépôt: 22.09.2003
(51) Int. Cl.: C07D 333/38

(54) **Nouveau procédé de synthèse industriel du ranélate de strontium et de ses hydrates**
Verfahren zur industriellen Herstellung von Strontiumranelat und seinen Hydraten
Process for the industrial synthesis of strontium ranelate and hydrates thereof

(30) Priorité: 24.09.2002 FR 0211763
(43) Date de publication de la demande: 31.03.2004
(73) Titulaire: Les Laboratoires Servier, 92284 Suresnes Cedex (FR)
(72) Inventeur: Vaysse-Ludot, Lucile, 76490 Saint Wandrille Rançon (FR); Lecouve, Jean-Pierre, 76600 Le Havre (FR); Langlois, Pascal, 76210 Saint Jean de la Neuville (FR)

(56) Documents cités:
- EP-A- 0 415 850
- EP-A- 0 813 869
- WIERZBICKI M. ET AL: "Réactivité des amino-2 thiophènes. Application à la synthèse de quelques thiéno[2,3-b]pyrroles" BULLETIN DE LA SOCIETÉ CHIMIQUE DE FRANCE, MASSON, PARIS, FR, no. 7-8, 1975, pages 1786-1792, XP009008611 ISSN: 0037-8968

## Description

La présente invention concerne un procédé de synthèse industriel du ranélate de strontium de formule (I) : 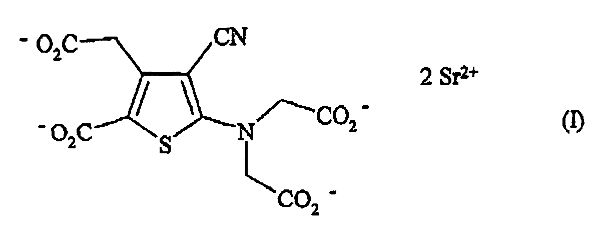 ou sel distrontique de l'acide 5-[bis(carboxyméthyl)amino]-3-carboxyméthyl-4-cyano-2-thiophènecarboxylique, et de ses hydrates, par réaction du tétraester correspondant avec l'hydroxyde de strontium au reflux de l'eau, selon la revendication 1.

Le ranélate de strontium possède des propriétés pharmacologiques et thérapeutiques très intéressantes, notamment des propriétés anti-ostéoporotiques remarquables, qui rendent ce composé utile dans le traitement des maladies osseuses.

Le ranélate de strontium, sa préparation et son utilisation en thérapeutique ont été décrits dans le brevet européen EP 0415 850.

Toutefois, la préparation industrielle d'un dérivé tel que le ranélate de strontium nécessite une étude approfondie de toutes les étapes réactionnelles, du choix des matières premières, des réactifs, et des solvants permettant d'obtenir les rendements optima.

La Demanderesse a mis au point un procédé de synthèse du ranélate de strontium de formule (I) dans lequel ces conditions ont été réunies par l'utilisation d'un ensemble de techniques et procédés particulièrement intéressants.

Le brevet EP 0415 850 décrit l'accès au ranélate de strontium à partir du tétraester éthylique de formule (IIa) : 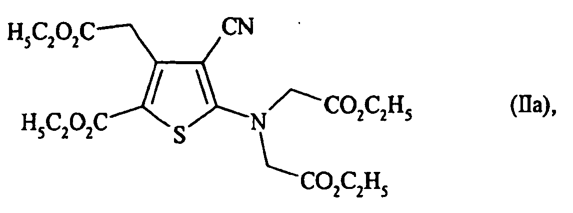 lui-même accessible à partir du diester éthylique de formule (IIIa) :

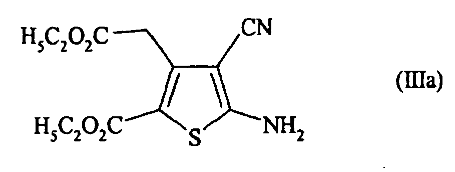

L'accès à l'intermédiaire de formule (IIIa) a été décrit dans la publication Bull. Soc. Chim. France 1975, pp. 1786-1792 et dans la publication J. Chem. Tech. Biotechnol. 1990, 47, pp. 39-46, par réaction entre le 3-oxoglutarate de diéthyle, le malononitrile et le soufre, dans l'éthanol, en présence de morpholine ou de diéthylamine.

Ce procédé présente l'avantage d'utiliser des matières premières aisément accessibles, et d'être simple à mettre en oeuvre, mais, transposé à l'échelle de quelques centaines de kg, il ne permet pas d'obtenir le composé de formule (IIIa) avec un rendement supérieur à 70 %.

La Demanderesse, pour synthétiser industriellement le ranélate de strontium de formule (I), a mis au point un procédé de synthèse industriel performant, permettant d'obtenir l'intermédiaire de formule (III) : 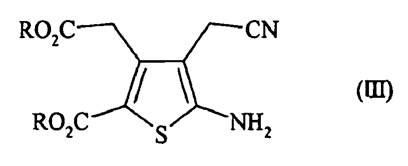 dans laquelle R représente un groupement alkyle (C₁-C₆) linéaire ou ramifié, avec une pureté supérieure à 97 % et un rendement au moins égal à 77 %, reproductible à l'échelle industrielle.

Plus spécifiquement, la synthèse industrielle du diester de formule (III), mise au point par la Demanderesse pour la synthèse industrielle du ranélate de strontium de formule (I), met en oeuvre comme matière première le composé de formule (IV) : 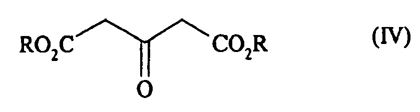 dans laquelle R est tel que défini précédemment,
que l'on met en réaction avec le malononitrile de formule (V) : 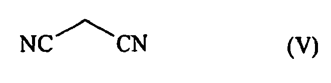 dans le méthanol,
en présence de morpholine en quantité supérieure à 0,95 mole par mole de composé de formule (IV),
pour conduire au composé de formule (VI) : 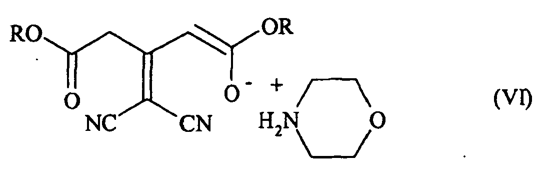 dans laquelle R est tel que défini précédemment,
que l'on met ensuite en réaction avec du soufre en quantité supérieure à 0,95 mole par mole de composé de formule (IV),
que l'on chauffe ensuite le mélange réactionnel au reflux,
et que l'on isole le composé de formule (III) ainsi obtenu par précipitation en présence d'eau, suivie d'une filtration.

Le procédé, ainsi amélioré par l'utilisation de ces conditions très spécifiques, et notamment par la formation intermédiaire du composé de formule (VI), éventuellement isolable, permet d'obtenir le composé de formule (III) avec une excellente pureté et un rendement reproductible à l'échelle de quelques centaines de kg et au moins égal à 77 %, ce qui représente un gain de rendement capital, compte-tenu des tonnages importants de ranélate de strontium produits.

De façon préférentielle, la quantité de méthanol est comprise entre 1 et 3 ml par gramme de composé de formule (IV).

La température de réaction entre les composés de formules (IV) et (V) est de préférence inférieure à 50°C.

Le temps de réaction au reflux après addition du soufre est préférentiellement compris entre 1 h 30 et 3 h.

La deuxième étape du procédé de synthèse industriel du ranélate de strontium de formule (I) mis au point par la Demanderesse consiste à transformer le composé de formule (III) en composé de formule (II) : 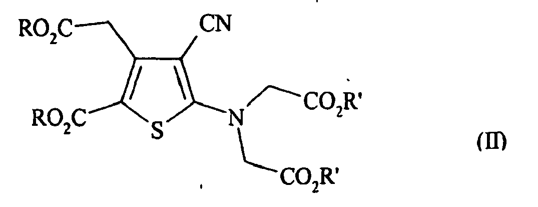 dans laquelle R est tel que défini précédemment, et R' représente un groupement alkyle (C₁-C₆) linéaire ou ramifié.

Le journal Bull. Soc. Chim. France 1975, pp. 1786-1792, décrit l'obtention du dérivé de formule (IIa), cas particulier des composés de formule (II) pour lequel R = R' = éthyle, par réaction de l'acide 5-amino-3-(carboxyméthyl)-4-cyano-2-thiophènecarboxylique avec le bromoacétate d'éthyle, en présence de carbonate de potassium, suivie d'un isolement en milieu hydro-organique très dilué.

Cependant, le faible rendement de cette réaction (65 %), la grande quantité de rejets aqueux salins générée par cette réaction, et surtout le temps de réaction très important (5 jours), étaient totalement dissuasifs quant à l'utilisation de cette réaction à l'échelle industrielle.

La Demanderesse, pour synthétiser industriellement le ranélate de strontium de formule (I), a mis au point un procédé de synthèse industriel simple, permettant d'obtenir le composé de formule (II) avec un très bon rendement, un temps de réaction considérablement plus court et une pureté excellente, et dans lequel les rejets aqueux salins sont complètement supprimés.

Plus spécifiquement, la synthèse industrielle du tétraester de formule (II), mise au point par la Demanderesse pour la synthèse du ranélate de strontium de formule (I), met en oeuvre le composé de formule (III) : 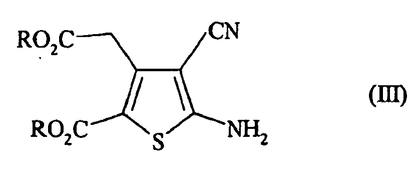 dans laquelle R représente un groupement alkyle (C₁-C₆) linéaire ou ramifié,
que l'on met en réaction avec un composé de formule (VII) : 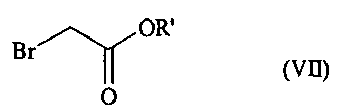 dans laquelle R' représente un groupement alkyle (C₁-C₆) linéaire ou ramifié,
en présence d'une quantité catalytique d'un ammonium quaternaire de type C₈-C₁₀,
et de carbonate de potassium,
au reflux d'un solvant organique,
que l'on filtre ensuite le mélange réactionnel,
puis que l'on concentre le milieu par distillation,
que l'on ajoute ensuite un cosolvant,
refroidit et filtre le mélange réactionnel,
pour conduire, après séchage de la poudre ainsi obtenue, au composé de formule (II).

Par ammonium quaternaire de type C₈-C₁₀, on entend un composé de formule (A) ou un mélange de composés de formule (A) :

R₁R₂R₃R₄-N^{+ -}X (A)

dans laquelle R₁ représente un groupement alkyle (C₁-C₆), R₂, R₃ et R₄, identiques ou différents, représentent chacun un groupement alkyle (C₈-C₁₀), et X représente un atome d'halogène.

Les ammoniums quaternaires de type C₈-C₁₀ préférés sont les catalyseurs Adogen 464® et Aliquat 336® .

De façon surprenante, seule l'utilisation d'un ammonium quaternaire de type C₈-C₁₀ permet l'obtention du composé de formule (II) à la fois avec un temps de réaction très réduit et avec une très bonne sélectivité, à la différence d'autres types d'ammoniums quaternaires, comme le montre le tableau suivant :

| **Catalyseur** | **Durée de réaction** | **Titre du milieu réactionnel** |
|---|---|---|
| Tétrabutylammoniumhydrogénosulfate (TBAHS) | 12 h | 92 % |
| Bromure de N,N-bis(2-hydroxyéthyl)-N-méthyl 1-dodécanaminium | 18 h | 82 % |
| Adogen 464® | 5 h | 96 % |
| Aliquat 336® | 4 h | 95 % |

De plus, l'isolement, pourtant simplifié (l'étape de précipitation suivie d'une filtration a été remplacée par une simple filtration du mélange réactionnel) permet, grâce aux conditions particulières qui ont été mises au point, d'obtenir le composé de formule (II), non seulement avec un très bon rendement (89 %), mais également avec une pureté excellente (supérieure à 98 %), et en supprimant la charge environementale que représentaient les rejets aqueux salins.
- La quantité de carbonate de potassium est préférentiellement comprise entre 2 et 3 moles par mole de composé de formule (III).
- La quantité de composé de formule (VII) est préférentiellement comprise entre 2 et 3 moles par mole de composé de formule (III).
- Le volume initial de solvant organique est préférentiellement compris entre 6 et 12 ml par gramme de composé de formule (III).
- Les solvants organiques préférés pour la réaction sont l'acétone et l'acétonitrile.
- Le cosolvant préféré pour l'isolement est le méthanol.

La troisième et dernière étape du procédé de synthèse industriel du ranélate de strontium de formule (I) mis au point par la Demanderesse consiste à transformer le tétraester de formule (II) en sel distrontique du tétracide correspondant.

Cette dernière étape est l'object principal de la présente invention.

Le brevet EP 0415 850 décrit trois méthodes pour cette transformation. La troisième des méthodes décrites, qui consiste à chauffer le composé de formule (IIa), cas particulier des composés de formule (II), en milieu hydroalcoolique, avec l'hydroxyde de strontium, puis à distiller l'éthanol et à isoler le composé de formule (I) par précipitation, présente l'avantage d'être extrêmement simple à mettre en oeuvre.

Cependant, en opérant dans les conditions décrites pour cette troisième méthode, la Demanderesse n'a obtenu le ranélate de strontium qu'avec un rendement de 80 % et une pureté de 87 %.

Or, le ranélate de strontium étant insoluble dans la plupart des solvants, sa purification ultérieure est extrêmement laborieuse. Une telle méthode était donc incompatible avec l'utilisation du ranélate de strontium comme principe actif pharmaceutique, qui nécessite une pureté supérieure ou égale à 98 %.

La Demanderesse a mis au point un procédé de synthèse industriel permettant l'obtention du ranélate de strontium, non seulement avec une pureté chimique excellente, ne nécessitant pas de retraitement avant son utilisation comme principe actif pharmaceutique, mais également avec un rendement excellent.

Plus spécifiquement, l'étape finale du procédé de synthèse industriel du ranélate de strontium de formule (I) mis au point par la Demanderesse met en oeuvre le composé de formule (II) : 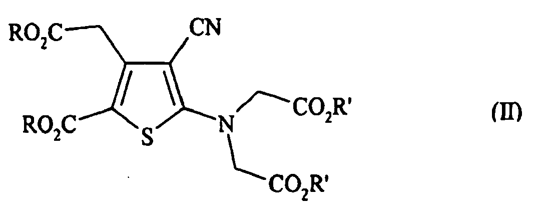 dans laquelle R et R', identiques ou différents, représentent chacun un groupement alkyle (C₁-C₆) linéaire ou ramifié, R représentant de préférence le groupement méthyle, et R' représentant de préférence un groupement méthyle ou éthyle,
que l'on met en réaction avec de l'hydroxyde de strontium en quantité supérieure ou égale à 2 moles par mole de composé de formule (II),
au reflux de l'eau,
pendant au moins 5 heures,
puis que l'on filtre le précipité obtenu à chaud,
que l'on lave le gâteau obtenu avec de l'eau bouillante,
pour conduire, après séchage de la poudre ainsi obtenue, au composé de formule (I) et à ses hydrates.
- De façon surprenante, le remplacement du mélange éthanol / eau par l'eau seule améliore de façon drastique, non seulement la pureté du ranélate de strontium obtenu, mais encore le rendement.
- D'autre part, la suppression de l'étape de distillation de l'éthanol simplifie encore le procédé.

La quantité d'eau dans le mélange réactionnel est préférentiellement supérieure ou égale à 8 ml par gramme de composé de formule (II).

La quantité d'hydroxyde de strontium est préférentiellement comprise entre 2 et 2,5 moles par mole de composé de formule (II).

Les exemples ci-dessous illustrent l'invention, mais ne la limitent en aucune façon.

Les exemples 1A et 1B illustrent la première étape du procédé de synthèse industriel du ranélate de strontium par la Demanderesse ; les exemples 2A, 2B, 2C et 2D, la deuxième étape de ce procédé ; enfin, l'exemple 3 illustre la troisième et dernière étape de ce procédé.

### EXEMPLE 1A : 5-Amino-4-cyano-3-(2-méthoxy-2-oxoéthyl)-2-thiophènecarboxylate de méthyle.

Charger dans un réacteur 400 kg de 3-oxoglutarate de diméthyle, 158 kg de malononitrile et 560 1 de méthanol, puis, en maintenant la température du milieu réactionnel inférieure à 40°C, 199,6 kg de morpholine.

Charger ensuite 73,6 kg de soufre, puis amener le mélange au reflux.

Après 2 h de réaction, couper le reflux, ajouter de l'eau jusqu'à précipitation. Filtrer le précipité obtenu, le laver et le sécher.

Le 5-amino-4-cyano-3-(2-méthoxy-2-oxoéthyl)-2-thiophènecarboxylate de méthyle est ainsi obtenu avec un rendement de 77 % et une pureté chimique de 98 %.

### EXEMPLE 1B: 5-Amino-4-cyano-3-(2-méthoxy-2-oxoéthyl)-2-thiophènecarboxylate de méthyle.

Charger dans un réacteur 400 kg de 3-oxoglutarate de diméthyle, 158 kg de malononitrile et 560 1 de méthanol, puis, en maintenant la température du milieu réactionnel inférieure à 40°C, 199,6 kg de morpholine.

Le composé de formule (VI) ainsi obtenu, ou sel d'addition du 3-(dicyanométhylène)-5-hydroxy-5-méthoxy-4-penténoate de méthyle avec la morpholine, est isolé par filtration après refroidissement du milieu, puis mis en réaction dans le méthanol avec 73,6 kg de soufre.

Le mélange est ensuite amené au reflux.

Après 2 h de réaction, couper le reflux, ajouter de l'eau jusqu'à précipitation. Filtrer le précipité obtenu, le laver et le sécher.

### EXEMPLE 2A: 5-[Bis(2-méthoxy-2-oxoéthyl)amino]-4-cyano-3-(2-méthoxy-2-oxoéthyl)-2-thiophènecarboxylate de méthyle.

Dans un réacteur, charger 400 kg de l'ester diméthylique de l'acide 5-amino-3-(carboxyméthyl)-4-cyano-2-thiophènecarboxylique, 478 kg de carbonate de potassium, 2810 1 d'acétone, 16 kg d'Adogen 464® et 529,6 kg de bromoacétate de méthyle.

Amener la température à 60°C. Après 5 h de reflux, refroidir le mélange réactionnel, puis le filtrer. Concentrer le filtrat obtenu.

Ajouter du méthanol, refroidir et filtrer la suspension obtenue, puis sécher la poudre.

Le 5-[bis(2-méthoxy-2-oxoéthyl)amino]-4-cyano-3-(2-méthoxy-2-oxoéthyl)-2-thiophène-carboxylate de méthyle est ainsi obtenu avec un rendement supérieur à 85 % et une pureté chimique supérieure à 98 %.

### EXEMPLE 2B: 5-[Bis(2-méthoxy-2-oxoéthyl)amino]-4-cyano-3-(2-méthoxy-2-oxoéthyl)-2-thiophènecarboxylate de méthyle.

Le 5-[bis(2-méthoxy-2-oxoéthyl)amino]-4-cyano-3-(2-méthoxy-2-oxoéthyl)-2-thiophène carboxylate de méthyle est obtenu de la même façon que dans l'exemple 2A, en remplaçant l'Adogen 464® par l'Aliquat 336®.

### EXEMPLE 2C: 5-[Bis(2-méthoxy-2-oxoéthyl)amino]-4-cyano-3-(2-méthoxy-2-oxoéthyl)-2-thiophènecarboxylate de méthyle.

Le 5-[bis(2-méthoxy-2-oxoéthyl)amino]-4-cyano-3-(2-méthoxy-2-oxoéthyl)-2-thiophène carboxylate de méthyle est obtenu de la même façon que dans l'exemple 2A, en remplaçant l'acétone par l'acétonitrile.

### EXEMPLE 2D: 5-[Bis(2-éthoxy-2-oxoéthyl)aminol-4-cyano-3-(2-méthoxy-2-oxoéthyl)-2-thiophènecarboxylate de méthyle.

Le 5-[bis(2-éthoxy-2-oxoéthyl)amino]-4-cyano-3-(2-méthoxy-2-oxoéthyl)-2-thiophène carboxylate de méthyle est obtenu de la même façon que dans l'exemple 2A, en remplaçant les 529,6 kg de bromoacétate de méthyle par 578,1 kg de bromoacétate d'éthyle.

### EXEMPLE 3 : Sel distrontique de l'acide 5-[bis(carboxyméthyl)amino]-3-carboxyméthyl-4-cyano-2-thiophènecarboxylique, octahydrate.

Charger dans un réacteur 770 kg d'hydroxyde de strontium et 5 500 l d'eau, puis 550 kg de 5-[bis(2-méthoxy-2-oxoéthyl)amino]-4-cyano-3-(2-méthoxy-2-oxoéthyl)-2-thiophène carboxylate de méthyle. Porter au reflux et maintenir le reflux pendant 5 heures minimum, puis filtrer le mélange réactionnel à chaud, laver le gâteau par de l'eau bouillante, et sécher la poudre obtenue.

L'octahydrate du sel distrontique de l'acide 5-[bis(carboxyméthyl)amino]-3-carboxyméthyl-4-cyano-2-thiophènecarboxylique est ainsi obtenu avec un rendement de 96% et une pureté chimique de 98 %.

## Revendications

1. Procédé de synthèse industriel du ranélate de strontium de formule (I) : 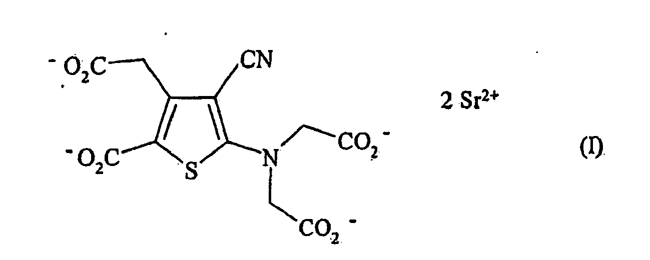 et de ses hydrates,
**caractérisé en ce que** l'on met en réaction le composé de formule (II) : 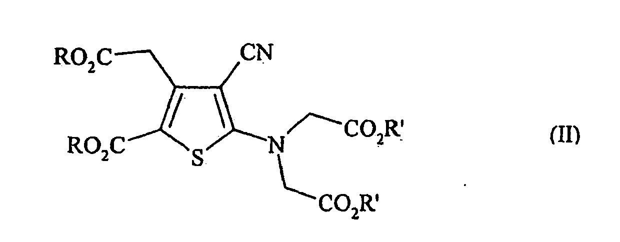 dans laquelle R et R', identiques ou différents, représentent chacun un groupement alkyle (C₁-C₆) linéaire ou ramifié,
avec de l'hydroxyde de strontium en quantité supérieure ou égale à 2 moles par mole de composé de formule (II),
au reflux de l'eau,
pendant au moins 5 heures,
puis que l'on filtre le précipité obtenu à chaud,
que l'on lave le gâteau obtenu avec de l'eau bouillante,
pour conduire, après séchage de la poudre ainsi obtenue, au composé de formule (I) et à ses hydrates.

2. Procédé de synthèse selon la revendication 1, **caractérisé en ce que** la quantité d'eau utilisée pour la réaction du composé de formule (II) avec l'hydroxyde de strontium est supérieure ou égale à 8 ml par gramme de composé de formule (II).

3. Procédé de synthèse selon l'une quelconque des revendications 1 ou 2, **caractérisé en ce que** la quantité d'hydroxyde de strontium est comprise entre 2 et 2,5 moles par mole de composé de formule (II).

4. Procédé de synthèse selon l'une quelconque des revendications 1 à 3, **caractérisé en ce que** R représente le groupement méthyle et R' représente un groupement méthyle ou éthyle.

5. Procédé de synthèse industriel du ranélate de strontium de formule (I) : 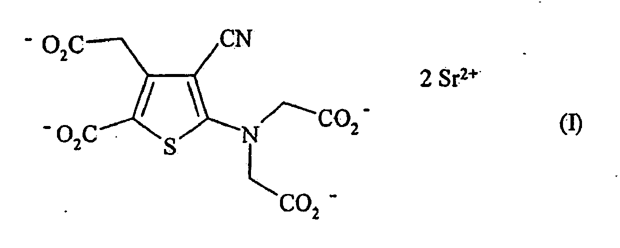 et de ses hydrates,
**caractérisé en ce que** l'on met en réaction le composé de formule (IV) : 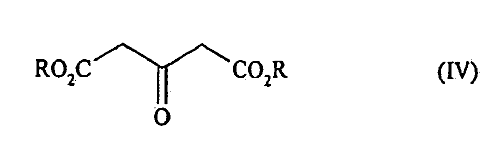 dans laquelle R représente un groupement alkyle (C₁-C₆) linéaire ou ramifié, avec le malononitrile de formule (V) : 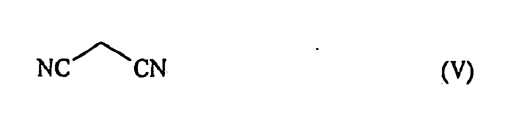 dans le méthanol,
en présence de morpholine en quantité supérieure à 0,95 mole par mole de composé de formule (IV),
pour conduire au composé de formule (VI) : 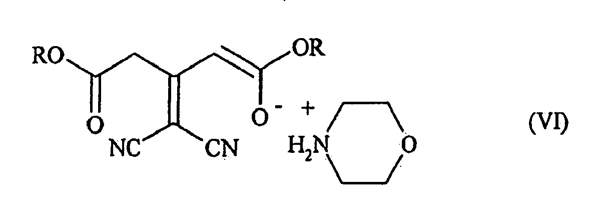 dans laquelle R est tel que défini précédemment,
que l'on met ensuite en réaction avec du soufre en quantité supérieure à 0,95 mole par mole de composé de formule (IV),
que l'on chauffe ensuite le mélange réactionnel au reflux,
et que l'on isole le composé ainsi obtenu par précipitation en présence d'eau, suivie d'une filtration,
pour conduire au composé de formule (III) : 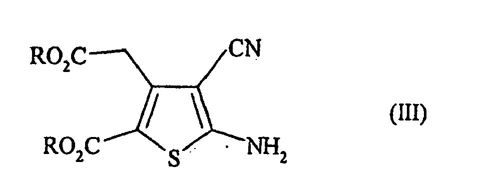 dans laquelle R est tel que défini précédemment,
que l'on met en réaction avec un composé de formule (VII) : 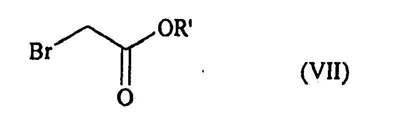 dans laquelle R' représente un groupement alkyle (C₁-C₆) linéaire ou ramifié,
en présence d'une quantité catalytique d'un ammonium quaternaire de type C₈-C₁₀, et de carbonate de potassium,
au reflux d'un solvant organique,
que l'on filtre ensuite le mélange réactionnel,
puis que l'on concentre le milieu par distillation,
que l'on ajoute ensuite un cosolvant,
refroidit et filtre le mélange réactionnel,
pour conduire, après séchage de la poudre ainsi obtenue, au composé de formule (II) : 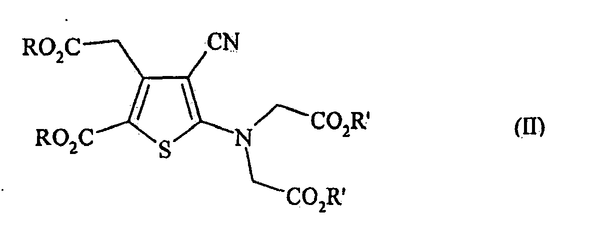 dans laquelle R et R' sont tels que définis précédemment,
que l'on transforme en composé de formule (I) ou en l'un de ses hydrates selon le procédé de l'une quelconque des revendications 1 à 4,
étant entendu que par ammonium quaternaire de type C₈-C₁₀, on entend un composé de formule (A) ou un mélange de composés de formule (A) :
R₁R₂R₃R₄-N⁺⁻X (A)
dans laquelle R₁ représente un groupement alkyle (C₁-C₆), R₂, R₃ et R₄, identiques ou différents, représentent chacun un groupement alkyle (C₈-C₁₀), et X représente un atome d'halogène.

6. Procédé de synthèse selon la revendication 5, **caractérisé en ce que** la quantité de méthanol utilisé dans la synthèse du composé de formule (III) est comprise entre 1 et 3 ml par gramme de composé de formule (IV).

7. Procédé de synthèse selon l'une quelconque des revendications 5 ou 6, **caractérisé en ce que** la température de réaction entre les composés de formule (IV) et (V) est inférieure à 50°C.

8. Procédé de synthèse selon l'une quelconque des revendications 5 à 7, **caractérisé en ce que** le temps de réaction au reflux entre le composé de formule (VI) et le soufre est compris entre 1 h 30 et 3 h.

9. Procédé de synthèse selon l'une quelconque des revendications 5 à 8, **caractérisé en ce que** la quantité de carbonate de potassium utilisé dans la synthèse du composé de formule (II) est comprise entre 2 et 3 moles par mole de composé de formule (III).

10. Procédé de synthèse selon l'une quelconque des revendications 5 à 9, **caractérisé en ce que** la quantité de composé de formule (VII) est comprise entre 2 et 3 moles par mole de composé de formule (III).

11. Procédé de synthèse selon l'une quelconque des revendications 5 à 10, **caractérisé en ce que** le volume initial de solvant organique utilisé pour la réaction du composé de formule (III) avec le composé de formule (VII) est compris entre 6 et 12 ml par gramme de composé de formule (III).

12. Procédé de synthèse selon l'une quelconque des revendications 5 à 11, **caractérisé en**
**ce que** le solvant organique utilisé pour la réaction du composé de formule (III) avec le composé de formule (VII) est l'acétone ou l'acétonitrile.

13. Procédé de synthèse selon l'une quelconque des revendications 5 à 12, **caractérisé en ce que** le cosolvant utilisé pour l'isolement du composé de formule (II) est le méthanol.

14. Procédé de synthèse selon l'une quelconque des revendications 5 à 13, **caractérisé en ce que** le composé de formule (II) obtenu a une pureté chimique supérieure à 98 %.

## Patentansprüche

1. Verfahren zur industriellen Synthese von Strontiumranelat der Formel (I): 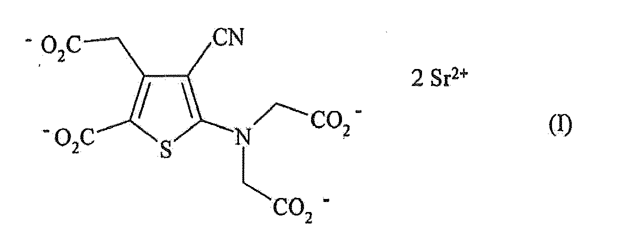 und von seinen Hydraten,
**dadurch gekennzeichnet, dass** man die Verbindung der Formel (II): 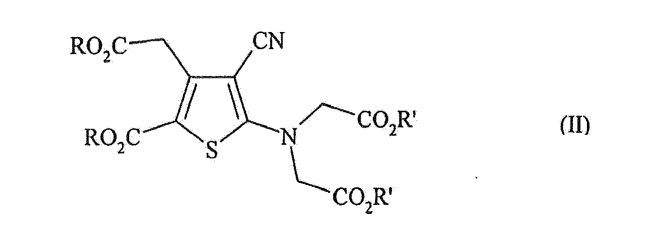 in der R und R', die identisch oder verschieden sind, jeweils eine geradkettige oder verzweigte (C₁-C₆)-Alkylgruppe bedeuten,
mit Strontiumhydroxid in einer Menge, die gleich oder größer ist als 2 Mol pro Mol der Verbindung der Formel (II),
am Rückfluss in Wasser,
während mindestens 5 Stunden umsetzt,
dann den erhaltenen Niederschlag in der Wärme filtriert und den erhaltenen Filterkuchen mit siedendem Wasser wäscht,
sodass man nach der Trocknung des in dieser Weise erhaltenen Pulvers die Verbindung der Formel (I) und ihre Hydrate erhält.

2. Syntheseverfahren nach Anspruch 1, **dadurch gekennzeichnet, dass** die für die Umsetzung der Verbindung der Formel (II) mit Strontiumhydroxid verwendete Wassermenge gleich oder größer ist als 8 ml pro Gramm der Verbindung der Formel (II).

3. Syntheseverfahren nach einem der Ansprüche 1 oder 2, **dadurch gekennzeichnet, dass** die Menge von Strontiumhydroxid zwischen 2 und 2,5 Mol pro Mol der Verbindung der Formel (II) beträgt.

4. Syntheseverfahren nach einem der Ansprüche 1 bis 3, **dadurch gekennzeichnet, dass** R die Methylgruppe und R' eine Methyl- oder Ethylgruppe bedeuten.

5. Verfahren zur industriellen Synthese von Strontiumranelat der Formel (I): 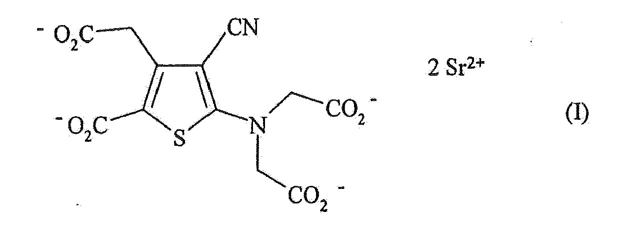 und von seinen Hydraten,
**dadurch gekennzeichnet, dass** man die Verbindung der Formel (IV): 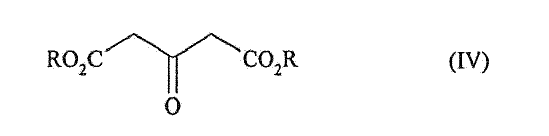 in der R eine geradkettige oder verzweigte (C₁-C₆)-Alkylgruppe bedeutet, mit Malononitril der Formel (V): 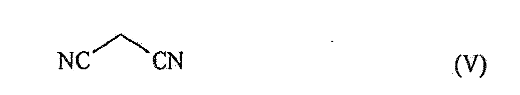 in Methanol
in Gegenwart von Morpholin in einer Menge von mehr als 0,95 Mol pro Mol der Verbindung der Formel (IV) umsetzt,
zur Bildung der Verbindung der Formel (VI): 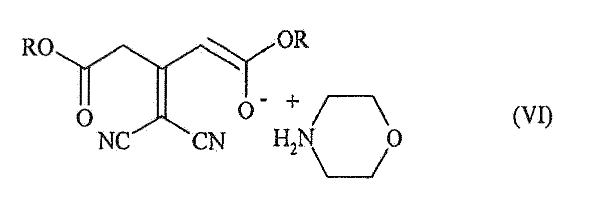 in der R die oben angegebenen Bedeutungen besitzt,
welche man anschließend mit Schwefel in einer Menge von mehr als 0,95 Mol pro Mol der Verbindung der Formel (IV) umsetzt,
man anschließend die Reaktionsmischung zum Sieden am Rückfluss erhitzt und die in dieser Weise erhaltene Verbindung durch Ausfällung in Gegenwart von Wasser, gefolgt von einer Filtration, isoliert,
sodass man die Verbindung der Formel (III) erhält: 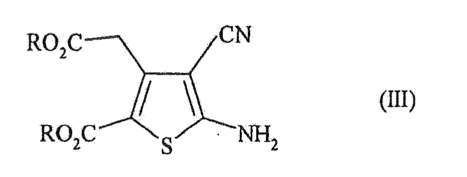 in der R die oben angegebenen Bedeutungen besitzt, welche man mit einer Verbindung der Formel (VII): 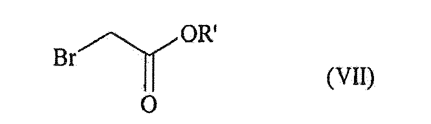 in der R' eine geradkettige oder verzweigte (C₁-C₆)-Alkylgruppe bedeutet,
in Gegenwart einer katalytischen Menge einer quaternären Ammoniumverbindung des Typs C₈-C₁₀
und Kaliumcarbonat,
am Rückfluss in einem organischen Lösungsmittel umsetzt,
anschließend die Reaktionsmischung filtriert,
dann das Medium durch Destillation einengt,
anschließend ein Co-Lösungsmittel zugibt,
die Reaktionsmischung abkühlt und filtriert,
sodass man nach dem Trocknen des in dieser Weise erhaltenen Pulvers die Verbindung der Formel (II) erhält: 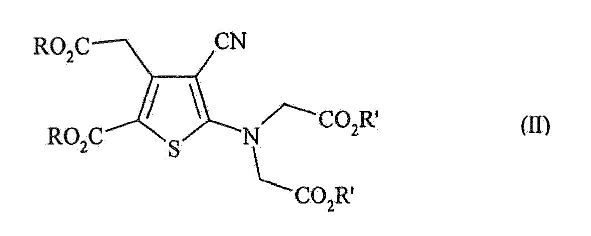 in der R und R' die oben angegebenen Bedeutungen besitzen,
welche man nach dem Verfahren gemäß einem der Ansprüche 1 bis 4 in die Verbindung der Formel (I) oder eines ihrer Hydrate umwandelt,
mit der Maßgabe, dass man unter der quaternären Ammoniumverbindung des Typs C₈-C₁₀ eine Verbindung der Formel (A) oder eine Mischung von Verbindungen der Formel (A) versteht:
R₁ R₂ R₃ R₄-N⁺⁻X (A)
in der R₁ eine (C₁-C₆)-Alkylgruppe, R₂, R₃ und R₄, die identisch oder verschieden sind, jeweils eine (C₈-C₁₀)-Alkylgruppe und X ein Halogenatom bedeuten.

6. Syntheseverfahren nach Anspruch 5, **dadurch gekennzeichnet, dass** die bei der Synthese der Verbindung der Formel (III) verwendete Methanolmenge zwischen 1 und 3 ml pro Gramm der Verbindung der Formel (IV) beträgt.

7. Syntheseverfahren nach einem der Ansprüche 5 oder 6, **dadurch gekennzeichnet, dass** die Temperatur der Reaktion der Verbindungen der Formeln (IV) und (V) weniger als 50 °C beträgt.

8. Syntheseverfahren nach einem der Ansprüche 5 bis 7, **dadurch gekennzeichnet, dass** die Dauer der Reaktion der Verbindung der Formel (VI) und Schwefel am Rückfluss zwischen 1 h 30 und 3 h beträgt.

9. Syntheseverfahren nach einem der Ansprüche 5 bis 8, **dadurch gekennzeichnet, dass** die bei der Synthese der Verbindung der Formel (II) verwendete Menge Kaliumcarbonat zwischen 2 und 3 Mol pro Mol der Verbindung der Formel (III) beträgt.

10. Syntheseverfahren nach einem der Ansprüche 5 bis 9, **dadurch gekennzeichnet, dass** die Menge der Verbindung der Formel (VII) zwischen 2 und 3 Mol pro Mol der Verbindung der Formel (III) beträgt.

11. Syntheseverfahren nach einem der Ansprüche 5 bis 10, **dadurch gekennzeichnet, dass** das Anfangsvolumen des für die Reaktion der Verbindung der Formel (III) mit der Verbindung der Formel (VII) verwendete organischen Lösungsmittels zwischen 6 und 12 ml pro Gramm der Verbindung der Formel (III) beträgt.

12. Syntheseverfahren nach einem der Ansprüche 5 bis 11, **dadurch gekennzeichnet, dass** das für die Reaktion der Verbindung der Formel (III) mit der Verbindung der Formel (VII) verwendete organische Lösungsmittel Aceton oder Acetonitril ist.

13. Syntheseverfahren nach einem der Ansprüche 5 bis 12, **dadurch gekennzeichnet, dass** das für die Isolierung der Verbindung der Formel (II) verwendete Co-Lösungsmittel Methanol ist.

14. Syntheseverfahren nach einem der Ansprüche 5 bis 13, **dadurch gekennzeichnet, dass** die erhaltene Verbindung der Formel (II) eine chemische Reinheit von mehr als 98 % aufweist.

## Claims

1. Process for the industrial synthesis of strontium ranelate of formula (I) : 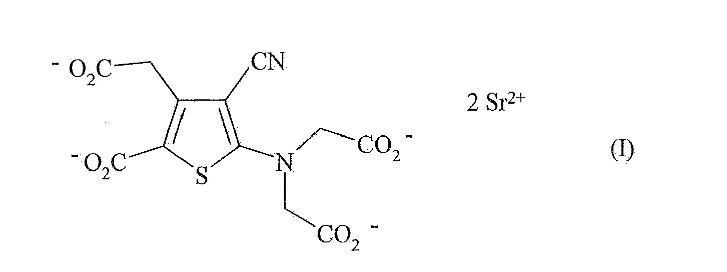 and its hydrates,
**characterised in that** the compound of formula (II) : 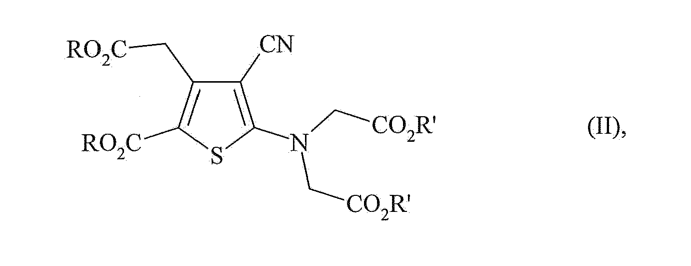 wherein R and R', which are the same or different, each represent a linear or branched (C₁-C₆)alkyl group,
is reacted with strontium hydroxide in an amount greater than or equal to 2 mol per mol of compound of formula (II),
at the reflux of water,
for at least 5 hours;
the precipitate obtained is then filtered off whilst hot;
the cake obtained is washed with boiling water
to yield, after drying of the powder thereby obtained, the compound of formula (I) and its hydrates.

2. Synthesis process according to claim 1, **characterised in that** the amount of water used in the reaction of the compound of formula (II) with strontium hydroxide is greater than or equal to 8 ml per gram of compound of formula (II).

3. Synthesis process according to either claim 1 or claim 2, **characterised in that** the amount of strontium hydroxide is from 2 to 2.5 mol per mol of compound of formula (II).

4. Synthesis process according to any one of claims 1 to 3, **characterised in that** R represents a methyl group and R' represents a methyl or ethyl group.

5. Process for the industrial synthesis of strontium ranelate of formula (I) : 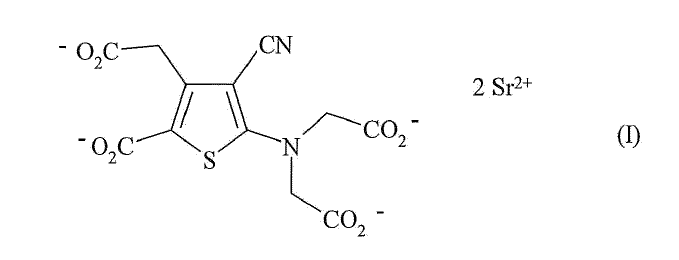 and its hydrates,
**characterised in that** the compound of formula (IV) : 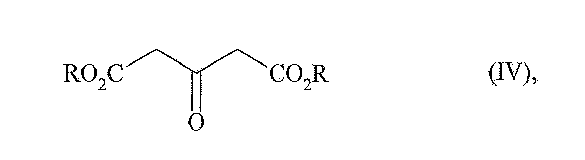 wherein R represents a linear or branched (C₁-C₆)alkyl group,
is reacted with malononitrile of formula (V) : 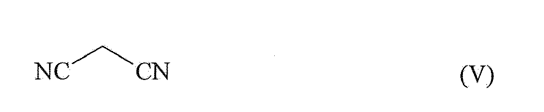 in methanol,
in the presence of morpholine in an amount greater than 0.95 mol per mol of compound of formula (IV),
to yield the compound of formula (VI) : 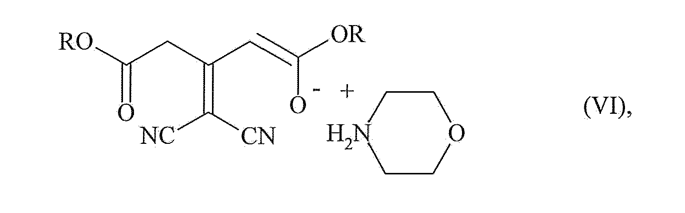 wherein R is as defined hereinbefore,
which is then reacted with sulphur in an amount greater than 0.95 mol per mol of compound of formula (IV);
the reaction mixture is then heated at reflux;
and the compound thereby obtained is isolated by precipitation in the presence of water, followed by filtration,
to yield the compound of formula (III) : 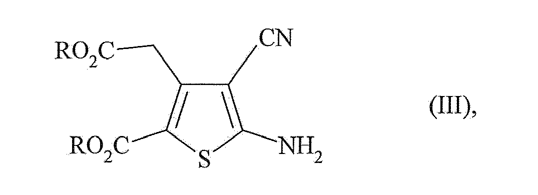 wherein R is as defined hereinbefore,
which is reacted with a compound of formula (VII) : 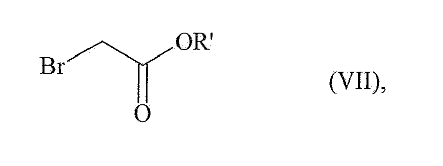 wherein R' represents a linear or branched (C₁-C₆)alkyl group,
in the presence of a catalytic amount of a C₈-C₁₀-type quaternary ammonium compound, and in the presence of potassium carbonate,
at the reflux of an organic solvent;
the reaction mixture is subsequently filtered;
the mixture is then concentrated by distillation;
a co-solvent is then added,
and the reaction mixture is cooled and filtered
to yield, after drying of the powder thereby obtained, the compound of formula (II) : 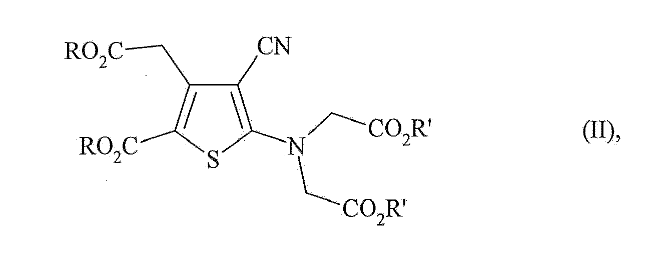 wherein R and R' are as defined hereinbefore,
which is converted into the compound of formula (I) or a hydrate thereof in accordance with the process of any one of claims 1 to 4,
it being understood that a C₈-C₁₀-type quaternary ammonium compound is a compound of formula (A) or a mixture of compounds of formula (A) :
R₁ R₂ R₃ R₄-N⁺⁻X (A)
wherein R₁ represents a (C₁-C₆)alkyl group, R₂, R₃ and R₄, which are the same or different, each represent a (C₈-C₁₀)alkyl group, and X represents a halogen atom.

6. Synthesis process according to claim 5, **characterised in that** the amount of methanol used in the synthesis of the compound of formula (III) is from 1 to 3 ml per gram of compound of formula (IV).

7. Synthesis process according to either claim 5 or claim 6, **characterised in that** the temperature of reaction between the compounds of formulate (IV) and (V) is less than 50°C.

8. Synthesis process according to any one of claims 5 to 7, **characterised in that** the refluxing time for the reaction between the compound of formula (VI) and sulphur is between 1 hour 30 minutes and 3 hours.

9. Synthesis process according to any one of claims 5 to 8, **characterised in that** the amount of potassium carbonate used in the synthesis of the compound of formula (II) is from 2 to 3 mol per mol of compound of formula (III).

10. Synthesis process according to any one of claims 5 to 9, **characterised in that** the amount of compound of formula (VII) is from 2 to 3 mol per mol of compound of formula (III).

11. Synthesis process according to any one of claims 5 to 10, **characterised in that** the initial volume of organic solvent used in the reaction of the compound of formula (III) with the compound of formula (VII) is from 6 to 12 ml per gram of compound of formula (III).

12. Synthesis process according to any one of claims 5 to 11, **characterised in that** the organic solvent used in the reaction of the compound of formula (III) with the compound of formula (VII) is acetone or acetonitrile.

13. Synthesis process according to any one of claims 5 to 12, **characterised in that** that the co-solvent used in the isolation of the compound of formula (II) is methanol.

14. Synthesis process according to any one of claims 5 to 13, **characterised in that** the compound of formula (II) obtained has a chemical purity greater than 98 %.
